(19)

Europäisches
Patentamt

European
Patent Office

Office européen
des brevets

(11)  **EP 3 165 155 B1**

(12)  **EUROPEAN PATENT SPECIFICATION**

(45) Date of publication and mention
of the grant of the patent:
**25.11.2020  Bulletin 2020/48**

(51) Int Cl.:
***A61B 5/021*** *(2006.01)*

(21) Application number: **16188011.7**

(22) Date of filing: **09.09.2016**

(54)  **METHOD AND APPARATUS FOR EXTRACTING FEATURE OF BIOSIGNAL**

VERFAHREN UND VORRICHTUNG ZUR MERKMALSEXTRAKTION VON BIOSIGNALEN

PROCÉDÉ ET APPAREIL PERMETTANT D'EXTRAIRE UNE CARACTÉRISTIQUE DE BIOSIGNAL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **09.11.2015  KR 20150156703**

(43) Date of publication of application:
**10.05.2017  Bulletin 2017/19**

(73) Proprietor: **Samsung Electronics Co., Ltd.
Suwon-si, Gyeonggi-do 16677 (KR)**

(72) Inventors:
• **YOON, Seung Keun**
**16678 Gyeonggi-do (KR)**
• **KWON, Ui Kun**
**16678 Gyeonggi-do (KR)**
• **KIM, Sang-Joon**
**16678 Gyeonggi-do (KR)**
• **PARK, Chang Soon**
**16678 Gyeonggi-do (KR)**
• **LEE, Jae Chun**
**16678 Gyeonggi-do (KR)**
• **CHOI, Chang Mok**
**16678 Gyeonggi-do (KR)**

(74) Representative: **Grünecker Patent- und
Rechtsanwälte
PartG mbB
Leopoldstraße 4
80802 München (DE)**

(56) References cited:
**US-A1- 2006 247 542     US-A1- 2013 079 656
US-A1- 2014 073 968**

**EP 3 165 155 B1**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

BACKGROUND

1. Field

The following description relates to a method and apparatus for extracting a feature of a biosignal.

2. Description of Related Art

Technology for monitoring a health of a user by analyzing a pulse waveform in a mobile sensing application, which utilizes a wearable device and various sensors have been provided. In a mobile sensing environment, various noises, in addition to a biosignal, may be produced or represented in a measurement of the biosignal according to a motion and a pose of a body. The noises may cause an error in analysing a biosignal waveform. Even when a small amount of noise is added to a basic waveform of a pulse wave, an intense distortion of a waveform may occur since an influence of the noise increases in response to the waveform being differentiated.

US 2014/0073968 A1 refers to methods and systems for qualifying physiological values based on two segments. A physiological monitoring system may process a physiological signal such a photoplethysmograph signal from a subject. The system may determine physiological information, such as a physiological rate, from the physiological signal. The system may use search techniques and qualification techniques to determine one or more initialization parameters. The initialization parameters may be used to calculate and qualify a physiological rate. The system may use signal conditioning to reduce noise in the physiological signal and to improve the determination of physiological information. The system may use qualification techniques to confirm determined physiological parameters. The system may also use autocorrelation techniques, cross-correlation techniques, fast start techniques, and/or reference waveforms when processing the physiological signal.

US 2013/0079656 A1 refers to systems and methods for determining respiration information from a photoplethysmograph. A signal representing physiological information may include information related to respiration. A patient monitoring system may generate a plurality of autocorrelation sequences from the signal and combine the autocorrelation sequences to generate a combined autocorrelation sequence. The combined autocorrelation sequence may be analysed to identify one or more peaks that may correspond to respiration information. Respiration information such as respiration rate may be determined based on the one or more peaks. US 2006/0247542 A1 refers to systems and methods for evaluating the degree of fatigue of a human body by using, as an index, change in waveform of a pulse wave, particularly acceleration pulse wave. Chaos analysis is performed on the acceleration pulse wave so that a degree of fatigue is evaluated by using, as an index, change of a correlation in the chaos analysis.

SUMMARY

It is the object of the present invention to provide an improved method and apparatus for extracting a feature of a biosignal.

This object is solved by the subject matter of the independent claims.

Preferred embodiments are defined by the dependent claims.

This Summary is provided to introduce a selection of concepts in a simplified form that are further described below in the Detailed Description. This Summary is not intended to identify key features or essential features of the claimed subject matter, nor is it intended to be used as an aid in determining the scope of the claimed subject matter.

In one general aspect, a method of extracting a feature of a biosignal is disclosed, the method including determining an acceleration waveform from a waveform of a biosignal, extracting an incident acceleration waveform from the acceleration waveform, and extracting a feature of the biosignal based on a cross-correlation between the acceleration waveform and the incident acceleration waveform.

The biosignal may include a pulse wave.

The determining of the acceleration waveform may include determining the acceleration waveform by performing a quadratic differential on the waveform.

The determining of the acceleration waveform may include determining the acceleration waveform based on an n-th sample value of the waveform, a (n-d)-th sample value of the waveform, and a (n+d)-th sample value of the waveform, wherein the d is a sample difference and the n is a positive integer greater than or equal to the d.

The method may further include at least one of acquiring a periodic waveform of the biosignal, or acquiring an average waveform of the biosignal.

The incident acceleration waveform may be an interval waveform corresponding to a pressure interval generating the biosignal.

The incident acceleration waveform may be an interval waveform corresponding to an interval during which an influence of a noise is minimal in comparison with a pulse wave signal generated by a heart contraction.

The extracting of the incident acceleration waveform may include extracting the incident acceleration waveform from the acceleration waveform based on a vascular reactivity index by a heart contraction pressure.

The extracting of the incident acceleration waveform may include extracting the incident acceleration waveform from an interval waveform from a maximum point of the acceleration waveform to a subsequent positive peak point.

The extracting of the feature of the biosignal may include calculating a degree of correlation between the incident acceleration waveform and the acceleration waveform over time while moving the incident acceleration waveform during an interval of the acceleration waveform.

The extracting of the feature of the biosignal may include extracting the feature of the biosignal based on at least one of a number of peak points included in the cross-correlation, positions of the peak points included in the cross-correlation, or values of the peak points included in the cross-correlation.

The biosignal may include a progressive wave starting from the heart and moving toward a body end portion and a reflective wave returned from the body end portion, and the extracting of the feature of the biosignal may include searching for an initial positive peak point among positive peak points greater than or equal to a degree of correlation included in the cross-correlation and extracting the initial positive peak point as a start point of the reflective wave.

In accordance with another general aspect, there is provided an apparatus for extracting a feature of a biosignal, the apparatus including a sensor configured to sense a biosignal, and a processor configured to determine an acceleration waveform from a waveform of the biosignal, extract an incident acceleration waveform from the acceleration waveform, and to extract a feature of the biosignal based on a cross-correlation between the acceleration waveform and the incident acceleration waveform.

The biosignal may include a pulse wave.

The processor may be configured to determine the acceleration waveform by performing a quadratic differential on the waveform.

The processor may be configured to determine the acceleration waveform based on an n-th sample value of the waveform, a (n-d)-th sample value of the waveform, and a (n+d)-th sample value of the waveform, and the d is a sample difference and the n is a positive integer greater than or equal to the d.

The processor may be configured to acquire a periodic waveform of the biosignal or acquire an average waveform of the biosignal.

The incident acceleration waveform may be an interval waveform corresponding to a pressure interval generating the biosignal.

The incident acceleration waveform may be an interval waveform corresponding to an interval during which an influence of a noise is minimal in comparison with a pulse wave signal generated by a heart contraction.

The processor may be configured to extract the incident acceleration waveform from the acceleration waveform based on a vascular reactivity index by a heart contraction pressure.

The processor may be configured to extract the incident acceleration waveform from an interval waveform from a maximum point of the acceleration waveform to a subsequent positive peak point.

The processor may be configured to calculate a degree of correlation between the incident acceleration waveform and the acceleration waveform over time while moving the incident acceleration waveform during an interval of the acceleration waveform.

The processor may be configured to extract the feature of the biosignal based on at least one of a number of peak points included in the cross-correlation, positions of the peak points included in the cross-correlation, or values of the peak points included in the cross-correlation.

The biosignal may include a progressive wave starting from the heart and moving toward a body end portion and a reflective wave returned from the body end portion, and the processor is configured to search for an initial positive peak point among positive peak points greater than or equal to a degree of correlation included in the cross-correlation and extract the initial positive peak point as a start point of the reflective wave.

In accordance with another general aspect, there is provided a method of extracting a feature of a signal, the method including obtaining an incident acceleration waveform from an acceleration waveform of the signal, and extracting a feature of the biosignal based on a cross-correlation between the acceleration waveform and the incident acceleration waveform.

The method may include determining the acceleration waveform by performing a quadratic differential on a pulse wave.

The method may include extracting the feature based on a search for an initial positive peak point having a cross-correlation greater than or equal to a degree of correlation.

The method may include extracting the feature based on a search for an initial negative peak point having a cross-correlation lesser than or equal to a degree of correlation.

The incident acceleration waveform may correspond to a portion of the acceleration waveform and the incident acceleration waveform may be an interval waveform corresponding to an interval during which an influence of a noise is minimal in comparison with a pulse wave signal generated by a heart contraction.

Other features and aspects will be apparent from the following detailed description, the drawings, and the claims.

BRIEF DESCRIPTION OF THE DRAWINGS

FIG. 1 illustrates an example of a sensed biosignal.
FIG. 2 is a diagram illustrating an example of a method of extracting a feature of a biosignal.
FIG 3 illustrates an example of a method of calculating an acceleration waveform.
FIG. 4 illustrates an example of a method of extracting an incident acceleration waveform.
FIG. 5 illustrates an example of a method of obtaining a cross-correlation between an acceleration waveform and an incident acceleration waveform.
FIG 6 illustrates an example of a method of extracting a feature of a biosignal based on a cross-correlation between an acceleration waveform and an incident acceleration waveform.
FIG. 7 is a diagram illustrating an example of a method of extracting a feature of a biosignal.
FIG. 8 is diagram illustrating an example of a result of extracting a feature of a biosignal.
FIG. 9 is a diagram illustrating an example of an apparatus for extracting a feature of a biosignal.

Throughout the drawings and the detailed description, unless otherwise described or provided, the same drawing reference numerals will be understood to refer to the same elements, features, and structures. The drawings may not be to scale, and the relative size, proportions, and depiction of elements in the drawings may be exaggerated for clarity, illustration, and convenience.

DETAILED DESCRIPTION

The following detailed description is provided to assist the reader in gaining a comprehensive understanding of the methods, apparatuses, and/or systems described herein. However, various changes, modifications, and equivalents of the methods, apparatuses, and/or systems described herein will be apparent to one of ordinary skill in the art after a full understanding of the present disclosure. The sequences of operations described herein are merely examples, and are not limited to those set forth herein, but may be changed as will be apparent to one of ordinary skill in the art, with the exception of operations necessarily occurring in a certain order. Also, descriptions of functions and constructions that are well known to one of ordinary skill in the art may be omitted for increased clarity and conciseness.

The features described herein may be embodied in different forms, and are not to be construed as being limited to the examples described herein. Rather, the examples described herein have been provided so that this disclosure will be thorough and complete, and will convey the full scope of the disclosure to one of ordinary skill in the art.

It will be understood that, although the terms first, second, etc. may be used herein to describe various elements, these elements should not be limited by these terms. These terms are only used to distinguish one element from another. For example, a first signal could be termed a second signal, and, similarly, a second signal could be termed a first signal without departing from the teachings of the disclosure.

It will be understood that when an element or layer is referred to as being "on", "attached to", or "connected to" another element or layer, it can be directly on or connected to the other element or layer or through intervening elements or layers may be present. In contrast, when an element is referred to as being "directly on", "directly attached to", or "directly connected to" another element or layer, there are no intervening elements or layers present. Other words used to describe the relationship between elements or layers should be interpreted in a like fashion (e.g., "between" versus "directly between," "adjacent" versus "directly adjacent," "on" versus "directly on").

The terminology used herein is for the purpose of describing particular examples only and not to limit the examples. As used herein, the singular forms "a", "an", and "the" are intended to include the plural forms as well, unless the context clearly indicates otherwise.

The following examples may be used for monitoring a health condition of a user. Examples may be implemented to monitor a health condition of a user in various forms, such as, for example, a personal computer, a laptop computer, a tablet computer, a mobile device, a smartphone, a television, a smart appliance, a smart vehicle, a wearable device, (such as, for example, a ring, a watch, a pair of glasses, glasses-type device, a bracelet, an ankle bracket, a belt, a necklace, an earring, a headband, a helmet, a device embedded in the cloths), a mobile device, a home appliance, content players, communication systems, image processing systems, graphics processing systems, or any other consumer electronics/information technology(CE/IT) device. The following examples may also be implemented smart home system, and may be applied to provide healthcare service for the user.

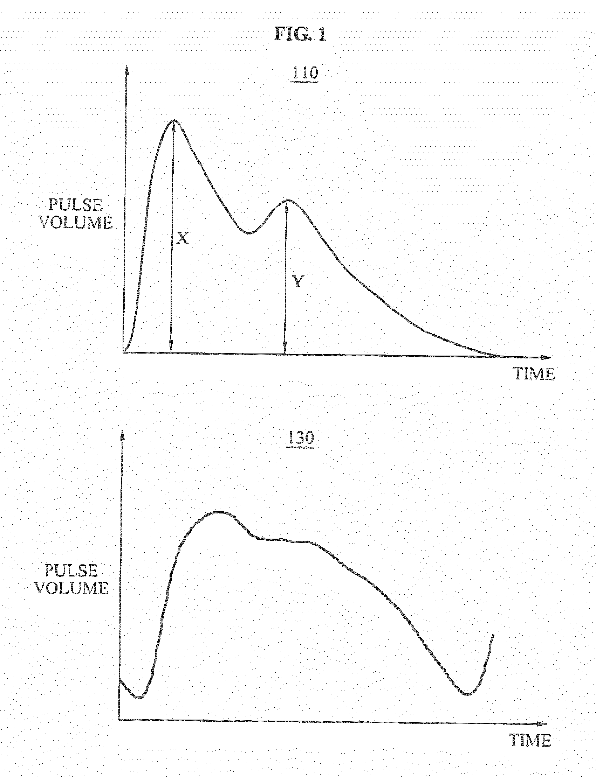

FIG. 1 illustrates an example of a sensed biosignal. Referring to FIG. 1, a basic waveform of a pulse wave is an example of a biosignal. For ease of description, a pulse wave is described as an example of a biosignal, but the biosignal is not limited thereto.

The pulse wave may be a pulsatory waveform appearing when blood is discharged from the heart and may be measured based on a change in blood flow and a change in vascular volume depending on a dilatation or contraction of the heart. In an example, an apparatus may include one or more sensors for photo-plethysmography (PPG) and use a light to observe features, for example, reflectivity, transmissivity, and absorptivity, with respect to a light of biological tissue appearing when the vascular volume is changed, so that the pulse wave may be measured based on the change. The pulse wave may be widely used since the pulse wave is a noninvasively measurable biosignal.

A waveform of the pulse wave includes a progressive wave X starting from the heart and moving toward a body end portion due to a heart contraction and a reflective wave Y returned from the body end portion. In an example, the progressive wave X overlaps the reflective wave Y. Waveforms generated due to elasticity of blood vessels may also overlap. The waveform of the pulse wave may have various forms based on an overlapping form of the progressive wave X and the reflective wave Y.

For example, as shown in a graph of a waveform 110, the progressive wave X and the reflective wave Y may overlap in a form in which positions of each of a start point and a positive peak point of the progressive wave X and the reflective wave Y are distinguishable. In another example, as shown in a graph of a waveform 130, the progressive wave X and the reflective wave Y may overlap in a form in which the positions of each of the start point and the positive peak point of the progressive wave X and the reflective wave Y are not distinguishable.

Various features obtained through searching the progressive wave X and the reflective wave Y may be used to monitor a health condition of a user. In an example, a blood pressure of the user may be estimated based on a time difference between the progressive wave X and the reflective wave Y. Thus, searching for a position of a start point of the reflective wave Y and a positive peak point of the reflective wave Y may be useful to analyze the pulse wave.

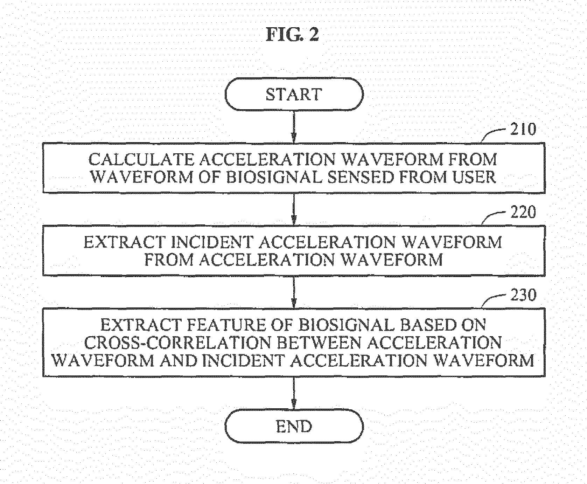

FIG. 2 is a diagram illustrating an example of a method of extracting a feature of a biosignal. The operations in FIG. 2 may be performed in the sequence and manner as shown, although the order of some operations may be changed or some of the operations omitted without departing from the spirit and scope of the illustrative examples described. Many of the operations shown in FIG. 2 may be performed in parallel or concurrently. In addition to the description of FIG. 2 below, the above descriptions of FIG. 1, are also applicable to FIG. 2, and are incorporated herein by reference. Thus, the above description may not be repeated here.

Referring to FIG. 2, in 210, an apparatus for extracting a feature of a biosignal, hereinafter referred to as an extracting apparatus, calculates an acceleration waveform from a waveform of the biosignal sensed from a user. In an example, the biosignal includes a pulse wave. The extracting apparatus acquires a periodic waveform of the biosignal or acquires an average waveform of the biosignal.

For example, the extracting apparatus may divide the waveform of the biosignal into a plurality of waveforms, each having start points and end points. The extracting apparatus may acquire a pulse waveform among the divided waveforms as a periodic waveform. In another example, the extracting apparatus may acquire the average waveform based on a method of acquiring an average waveform of each waveform corresponding to a unit length of an identical period.

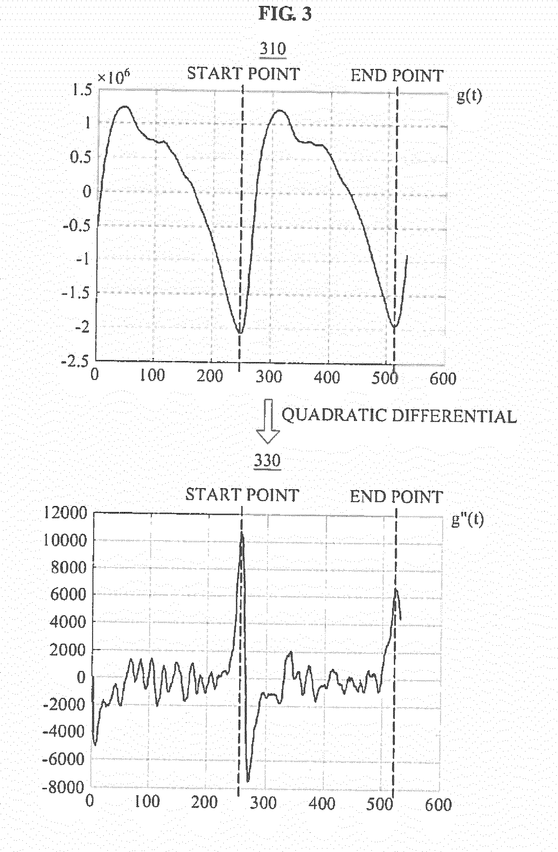

In an example, the extracting apparatus calculates the acceleration waveform by performing a quadratic differential on the waveform of the biosignal, for example. For example, the extracting apparatus calculates the acceleration waveform based on an n-th sample value of the waveform, a (n-d)-th sample value of the waveform, and a (n+d)-th sample value of the waveform, where "d" is a sample difference and "n" is a positive integer greater than or equal to the "d." A method of extracting the acceleration waveform will be described with reference to FIG. 3.

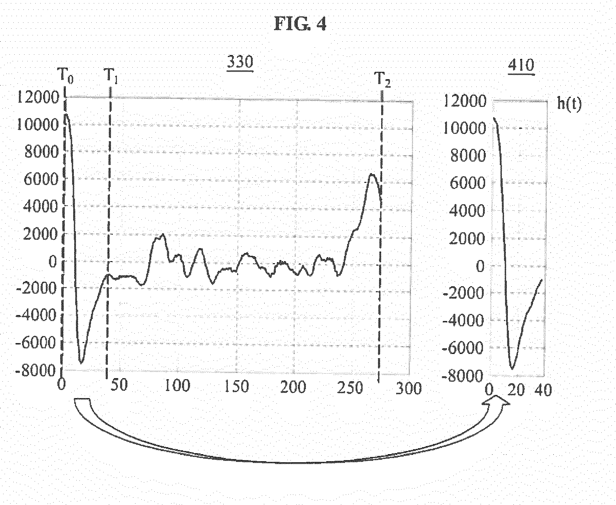

In 220, the extracting apparatus extracts an incident acceleration waveform from the acceleration waveform. The incident acceleration waveform corresponds to a portion of the acceleration waveform, and the incident acceleration waveform is an interval waveform corresponding to a pressure interval generating the biosignal. The incident acceleration waveform corresponds to the portion of the acceleration waveform and the incident acceleration waveform is an interval waveform corresponding to an interval during which an influence of a noise in comparison with a pulse wave signal generated by a heart contraction is smallest. A method of extracting the incident acceleration waveform will be described with reference to FIG. 4.

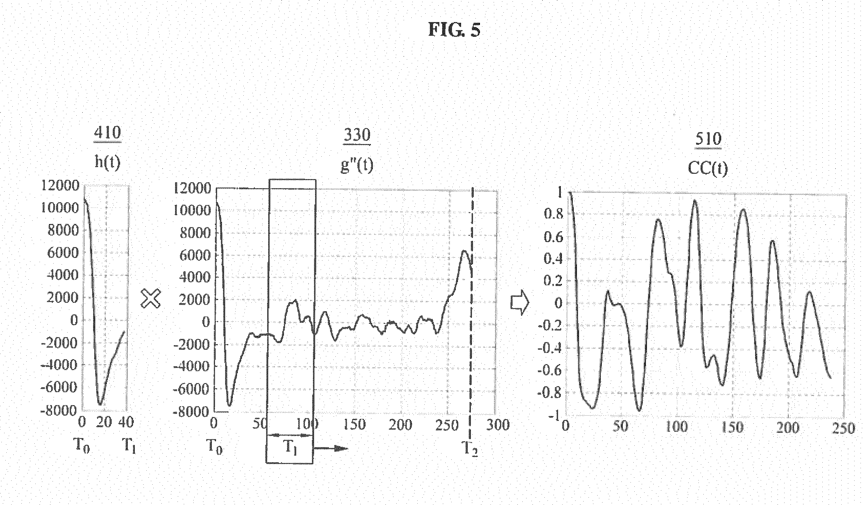

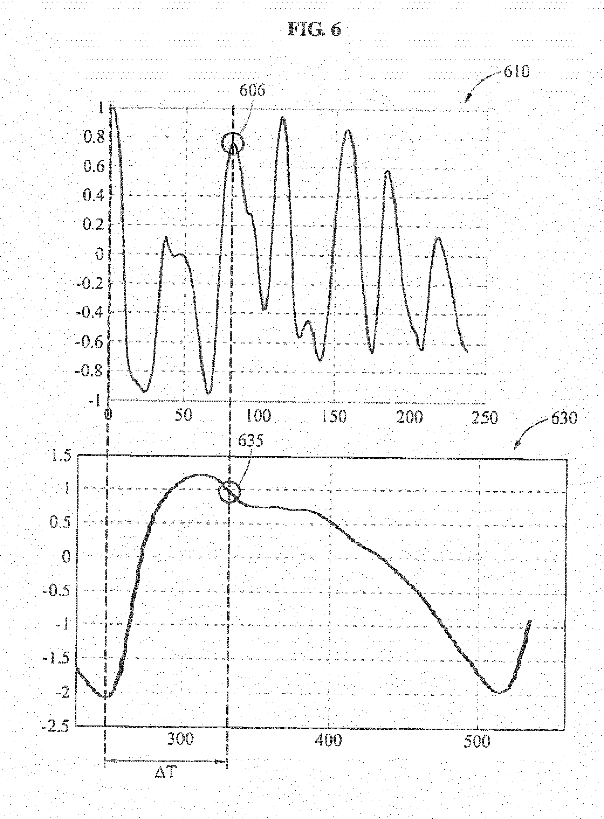

In 230, the extracting apparatus extracts the feature of the biosignal based on a cross-correlation between the acceleration waveform and the incident acceleration waveform. The extracting apparatus calculates a degree of correlation between the acceleration waveform and the incident acceleration waveform over time while moving the incident acceleration waveform during an interval of the acceleration waveform. The extracting apparatus may search for an initial, for example, positive peak point among positive peak points greater than or equal to a degree of correlation included in the cross-correlation and extract the positive peak point as a feature, for example, a start point of a reflective wave of the biosignal. In an example, the extracting apparatus may search for an initial negative peak point among negative peak points less than the degree of correlation included in the cross-correlation, and extract the negative peak point as the feature of the biosignal. A method of extracting the feature of the biosignal by the extracting apparatus will be described with reference to FIGS. 5 and 6.

FIG. 3 illustrates an example of a method of calculating an acceleration waveform. Referring to FIG. 3, a basic waveform g (t) 310 of a pulse wave and an acceleration waveform g"(t) 330 are calculated from the basic waveform 310.

The basic waveform 310 may be a waveform of a period of a pulse, for example, a waveform between a start point and an end point in the basic waveform 310. An extracting apparatus may calculate the acceleration waveform 330 by performing a quadratic differential on the basic waveform 310, for example. In an example, the acceleration waveform 330 may be an acceleration pulse waveform.

For example, in response to an n-th sample of the basic waveform 310 being $t_n$, the extracting apparatus may calculate the acceleration waveform 330 using Equation 1.

[Equation 1]

$$g''(t_n) = (g(t_{n+d}) - g(t_n)) - (g(t_n) - g(t_{n-d}))$$

$$= g(t_{n+d}) + g(t_{n-d}) - 2g(t_n)$$

In Equation 1, d is a positive number and denotes a sample difference determinable by a user based on a sampling rate and a noise of the basic waveform 310. The sample difference "d" may be determined in advance, and "n" is an integer greater than or equal to the "d."

In an example, the extracting apparatus may be a filter, which removes a noise amplified by performing the quadratic differential on the basic waveform 310 of the pulse wave.

Referring to FIG. 3, a portion in which an external pressure is strongest in a periodic waveform of the pulse wave is in a vicinity of a start point of the pulse wave, while a surrounding noise may be smallest at a start point of the acceleration waveform 330.

An incident acceleration waveform, which corresponds to a portion to which an initial pressure of the acceleration waveform 330 is applied, may be extracted to minimize the influence of the noise and to analyze a waveform in more detail. A method of extracting an incident acceleration waveform will be described with reference to FIG. 4.

FIG. 4 illustrates an example of a method of extracting an incident acceleration waveform. Referring to FIG. 4, an incident acceleration waveform h(t) 410 is extracted from the acceleration waveform g"(t) 330 .

The extracting apparatus may perform a prior search for a point, hereinafter referred to as a maximum point $T_0$, at which an acceleration waveform is maximized in a vicinity of the start point of the acceleration waveform 330 to calculate the incident acceleration waveform 410. The start point of the acceleration waveform 330 may correspond to a portion at which a heart contraction starts. The start point of the acceleration waveform 330 may correspond to an interval of which an amount of noise is small, as a start interval of a progressive wave.

The extracting apparatus may search for a position of a positive peak point $T_1$ positioned subsequent to the maximum point $T_0$ of the acceleration waveform 330. The extracting apparatus may extract the incident acceleration waveform 410 from an interval waveform from the maximum point $T_0$ of the acceleration waveform 330 to the subsequent positive peak point $T_1$ based on, for example, h(t) = g"(t) ($T_0 \leq t \leq T_1$). In another example, the extracting apparatus may extract an incident acceleration waveform from an acceleration waveform based on a vascular reactivity index indicating a cross-correlation between a heart contraction and a vascular elastance.

FIG. 5 illustrates an example of a method of obtaining a cross-correlation between an acceleration waveform and an incident acceleration waveform. Referring to FIG. 5, a graph 510 represents a cross-correlation between the incident acceleration waveform h(t) 410 and the acceleration waveform g"(t) 330.

In response to an end point of a pulse wave g(t) being $T_2$, the extracting apparatus may calculate the cross-correlation between the incident acceleration waveform h(t) 410 and the acceleration waveform g"(t) 330 based on a cross-correlation function CC(t) as shown in Equation 2. The cross-correlation may be a moving correlation or a consecutive cross-correlation between an acceleration waveform and an incident acceleration waveform.

[Equation 2]

$$CC(t) = \frac{\sum_{T=T_0}^{T_1} \{(g''(t+T) - \overline{g''(t+T)}) \cdot (h(T)-\overline{h(T)})\}}{\sqrt{\sum_{T=T_0}^{T_1} (g''(t+T) - \overline{g''(t+T)})^2 \cdot \sum_{T=T_0}^{T_1} (h(T) - \overline{h(T)})^2}} \quad (0 \leq t \leq T_2 - T_1)$$

In Equation 2, each value of $\overline{g''(t+T)}$ and $h(\overline{T})$ may be an average value in a range of $T_0 \leq T \leq T_1$.

The extracting apparatus may calculate the cross-correlation between the incident acceleration 410 and the acceleration waveform 330 over time while moving the incident acceleration waveform 410 during an interval of the acceleration waveform 330. For example, the extracting apparatus may calculate the cross-correlation over time while moving the incident acceleration waveform 410 from $T_0$ to $T_2$ of the acceleration waveform 330 by a width of $T_1$.

Each point of the graph 510 may correspond to a degree of a correlation between the incident acceleration waveform 410 and the acceleration waveform 330 calculated over time. In an example, the extracting apparatus may amplify a feature of the incident acceleration waveform 410 by calculating the degree of correlation over time while moving the incident acceleration waveform 410 during the interval of the acceleration waveform 330.

The extracting apparatus may obtain information through each peak point represented in the cross-correlation as shown in the graph 510. For example, the extracting apparatus extracts a feature of a biosignal based on a number of the peak points included in the cross-correlation, positions of the peak points included in the cross-correlation, and values of the peak points included in the cross-correlation. A method of extracting a feature of a biosignal based on a cross-correlation will be described with reference to FIG. 6.

FIG. 6 illustrates an example of a method of extracting a feature of a biosignal based on a cross-correlation between an acceleration waveform and an incident acceleration waveform. Referring to FIG. 6, a feature of a biosignal, for example, a pulse wave 630, is extracted from a cross-correlation 610.

The extracting apparatus may search for an initial positive peak point among positive peak points greater than or equal to a degree of correlation in the cross-correlation 610. For example, in response to the degree of correlation being set to 0.4, the extracting apparatus may search for a positive peak point 606 as the initial positive peak point. Since a degree of correlation at a start point of the cross-correlation 610 is 1, the start point may be excluded from positive peak points.

The initial positive peak point 606 found in the cross-correlation 610 may be a point at which a similarity with a start interval of a progressive wave is highest and may be estimated as a start interval of a reflective wave. In an example, the extracting apparatus may measure a blood pressure of a user by estimating a time difference between the progressive wave and the estimated reflective wave.

The extracting apparatus may search for a point 635 corresponding to the positive peak point 606 in the pulse wave 630, and extract the point 635 as a start point of the reflective wave at which a pressure of the reflective wave starts in the pulse wave 630.

For example, a feature to be obtained, by the extracting apparatus, through a peak point based on photoplethysmography (PPG) is as follows.

A first peak point indicates a start point of a pulse wave, and each of remaining peak points indicates a point at which a reflective wave arrives. Thus, when a number of peak points increases or an interval between the peak points decreases, a degree of vascular elasticity may increase.

Thus, the extracting apparatus may extract a feature of a biosignal based on the number of the peak points included in a cross-correlation using the aforementioned method.

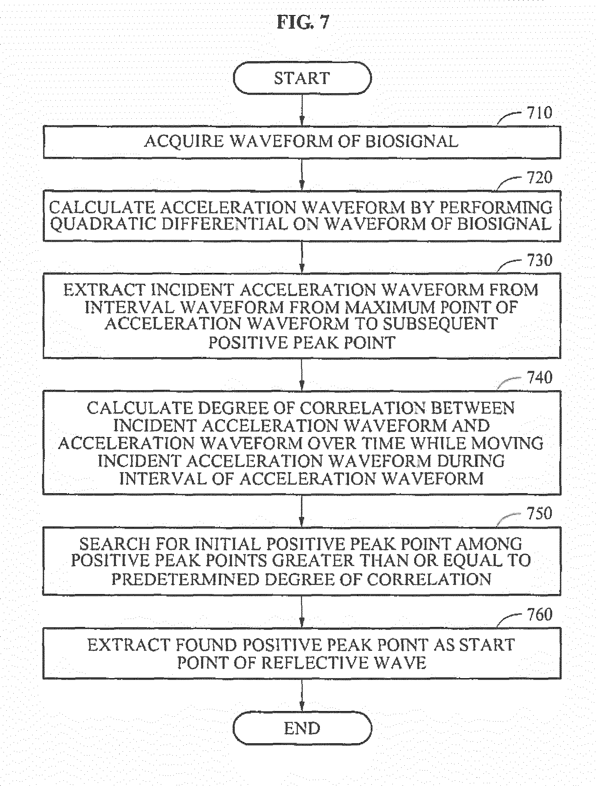

FIG. 7 is a diagram illustrating an example of a method of extracting a feature of a biosignal. The operations in FIG. 7 may be performed in the sequence and manner as shown, although the order of some operations may be changed or some of the operations omitted without departing from the spirit and scope of the illustrative examples described. Many of the operations shown in FIG. 7 may be performed in parallel or concurrently. In addition to the description of FIG. 7 below, the above descriptions of FIGS. 1-6, are also applicable to FIG. 7, and are incorporated herein by reference. Thus, the above description may not be repeated here.

In 710, an extracting apparatus acquires a waveform of a biosignal. In 720, the extracting apparatus calculates an acceleration waveform by performing a quadratic differential on the waveform of the biosignal, for example.

In 730, the extracting apparatus extracts an incident acceleration waveform from an interval waveform from a maximum point of the acceleration waveform to a subsequent positive peak point.

In 740, the extracting apparatus calculates a degree of correlation between the incident acceleration waveform and the acceleration waveform over time while moving the incident acceleration waveform during an interval of the acceleration waveform.

In 750, the extracting apparatus searches for an initial, for example, positive peak point among positive peak points greater than or equal to a degree of correlation included in a cross-correlation. In an example, the degree of correlation may be predetermined.

In 760, the extracting apparatus extracts the positive peak point as a start point of a reflective wave.

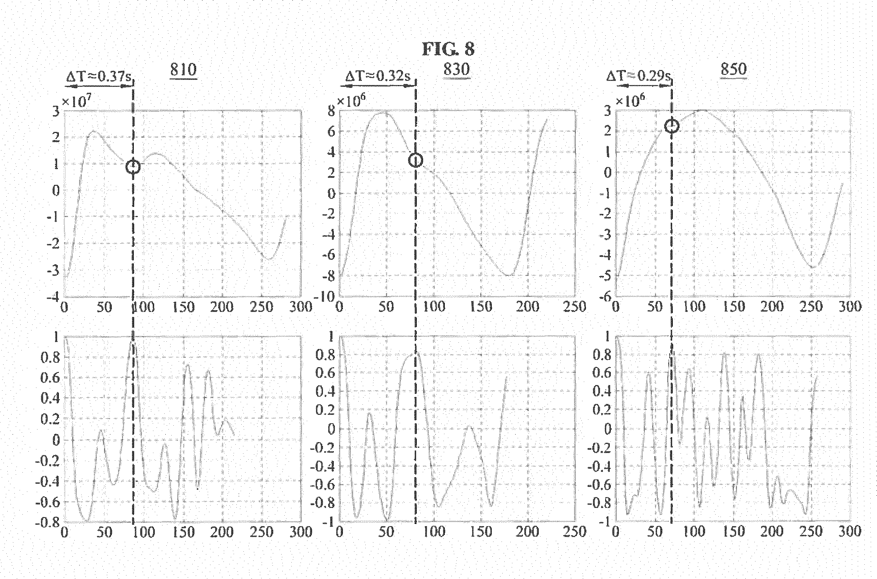

FIG. 8 is diagram illustrating an example of a result of extracting a feature of a biosignal using a method of extracting a feature of a biosignal. Referring to FIG. 8, the graphs show an extracting result 810 when a pressure, for example, a blood pressure, of a biosignal is 93/68 millimeter of mercury (mmHg), an extracting result 830 when the pressure of the biosignal is 104/78 mmHg, and an extracting result 850 when the pressure is 105/71 mmHg.

A time difference $\Delta T$ between a progressive wave and a reflective wave may be determined to be 0.37s by a start point of the reflected wave found in the extracting result 810. The time difference $\Delta T$ may be determined to be 0.32s by a start point of a reflective wave found in the extracting result 830, and the time difference $\Delta T$ may be determined to be 0.29s by a start point of the reflected wave found in the extracting result 850.

In an example, a start point of a reflective wave may also be extracted with respect to various pulse waveforms of which a difference between a progressive wave and a reflective wave is not distinguishable. In another example, an incident acceleration waveform which is an interval waveform corresponding to an interval during which an influence of a noise in comparison with a pulse wave signal by a heart contraction is smallest, thereby extracting a feature of a biosignal with respect to a noise in a robust manner.

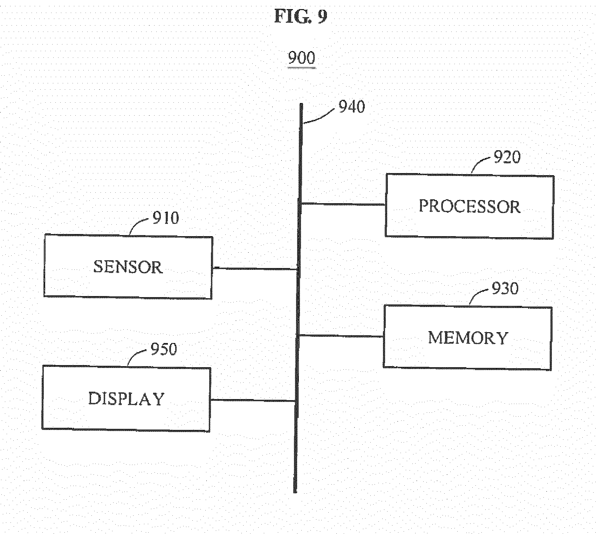

FIG. 9 is a diagram illustrating an example of an apparatus for extracting a feature of a biosignal. Referring to FIG. 9, an extracting apparatus 900 includes a sensor 910, a processor 920, a memory 930, and a display 950. In an example, the sensor 910, the processor 920, and the memory 930 may communicate with each other through a bus 940.

The sensor 910 senses a biosignal from a user. The biosignal includes a progressive wave starting from the heart and moving toward a body end portion and a reflective wave returned from the body end portion. The biosignal includes a pulse wave. The sensor 910 includes, for example, a PhotoPlethymoGraph (PPG) sensor. The PPG sensor may be a pulse wave measurement sensor to estimate a cardiac activity condition by measuring an amount of blood flowing in blood veins using an optical feature of a biological tissue.

The processor 920 calculates an acceleration waveform from a waveform of the biosignal and extracts an incident acceleration waveform from the acceleration waveform. The processor 920 acquires a periodic waveform of the biosignal or acquires an average waveform of the biosignal.

The incident acceleration waveform corresponds to a portion of the acceleration waveform, and the incident acceleration waveform is an interval waveform corresponding to a pressure interval generating the biosignal. The incident acceleration waveform corresponds to the portion of the acceleration waveform, and the incident acceleration waveform is an interval waveform corresponding to an interval during which an influence of a noise in comparison with a pulse wave signal by a heart contraction is smallest.

The processor 920 calculates the acceleration waveform by performing a quadratic differential on the waveform. The processor 920 calculates the acceleration waveform based on an n-th sample value of the waveform, a (n-d)-th sample value of the waveform, and a (n+d)-th sample value of the waveform. The "d" is a sample difference and the "n" is a positive integer greater than or equal to the "d."

The processor 920 extracts the feature of the biosignal based on a cross-correlation between the acceleration waveform and the incident acceleration waveform.

The processor 920 extracts the incident acceleration waveform from an interval waveform from a maximum point of the acceleration waveform to a subsequent positive peak point. The processor 920 calculates a degree of correlation between the incident acceleration waveform and the acceleration waveform over time while moving the incident acceleration waveform during an interval of the acceleration waveform.

The processor 920 extracts the feature of the biosignal based on at least one of a number of peak points included in the cross-correlation, positions of the peak points included in the cross-correlation, and values of the peak points included in the cross-correlation. The processor 920 searches for an initial positive peak point among positive peak points greater than or equal to a degree of correlation included in the cross-correlation and extracts the found positive peak point as a start point of the reflective wave. In an example, the processor 920 may determine for example, a blood pressure of the user, and may output the sensed blood pressure to a display 950 of the extracting apparatus 900.

A display 950 may be a physical structure that includes one or more hardware components that provide the ability to render a user interface and/or receive user input. The display 950 can encompass any combination of display region, gesture capture region, a touch sensitive display, and/or a configurable area. The display 950 can be embedded in the extracting apparatus 900 or may be an external peripheral device that may be attached and detached from the extracting apparatus 900. The display 950 may be a single-screen or a multi-screen display. A single physical screen can include multiple displays that are managed as separate logical displays permitting different content to be displayed on separate displays although part of the same physical screen. The display 950 may also be implemented as an eye glass display (EGD), which includes one-eyed glass or two-eyed glasses.

The memory 930 stores the acceleration waveform and the incident acceleration waveform calculated by the processor 920. The memory 930 stores the feature of the biosignal extracted by the processor 920.

In an example, the processor 920 may perform one or more of the methods described with reference to FIGS. 1 through 8.

The processor 920 executes a program and controls the extracting apparatus 900. A program code executed by the processor 920 is stored in the memory 930. The extracting apparatus 900 may be connected to an external device, for example, a personal computer or a network, through an input and output device (not shown), and may exchange data.

The memory 930 may be a volatile memory or a non-volatile memory.

In an example, the extracting apparatus 900 may be provided as a software module to be driven by at least one processor. The software module may be recorded, in a program form, in a memory connected to a processor. In another example, the extracting apparatus 900 may be a hardware module.

As a non-exhaustive illustration only, the extracting apparatus 900 may refer to mobile devices such as, for example, a mobile phone, a cellular phone, a smart phone, a wearable smart device (such as, for example, a ring, a watch, a pair of glasses, glasses-type device, a bracelet, an ankle bracket, a belt, a necklace, an earring, a headband, a helmet, a device embedded in the cloths), a personal computer (PC), a laptop, a notebook, a subnotebook, a netbook, or an ultra-mobile PC (UMPC), a tablet personal computer (tablet), a phablet, a mobile internet device (MID), a personal digital assistant (PDA), an enterprise digital assistant (EDA), a digital camera, a digital video camera, a portable game console, an MP3 player, a portable/personal multimedia player (PMP), a handheld e-book, an ultra mobile personal computer (UMPC), a portable lab-top PC, a global positioning system (GPS) navigation, a personal navigation device or portable navigation device (PND), a handheld game console, an e-book, and devices such as a high definition television (HDTV), an optical disc player, a DVD player, a Blue-ray player, a setup box, robot cleaners, a home appliance, content players, communication systems, image processing systems, graphics processing systems, other consumer electronics/information technology(CE/IT) device, or any other device capable of wireless communication or network communication consistent with that disclosed herein. The mobile device may be implemented as a smart appliance, an intelligent vehicle, or in a smart home system.

The mobile device may also be implemented as a wearable device, which is worn on a body of a user. In one example, a wearable device may be self-mountable on the body of the user, such as, for example, a watch, a bracelet, or as an eye glass display (EGD), which includes one-eyed glass or two-eyed glasses. In another non-exhaustive example, the wearable device may be mounted on the body of the user through an attaching device, such as, for example, attaching a smart phone or a tablet to the arm of a user using an armband, incorporating the wearable device in a cloth of the user, or hanging the wearable device around the neck of a user using a lanyard.

The apparatuses, units, modules, devices, and other components illustrated that perform the operations described herein are implemented by hardware components. Examples of hardware components include controllers, sensors, generators, drivers, and any other electronic components known to one of ordinary skill in the art. In one example, the hardware components are implemented by one or more processors or computers. A processor or computer is implemented by one or more processing elements, such as an array of logic gates, a controller and an arithmetic logic unit, a digital signal processor, a microcomputer, a programmable logic controller, a field-programmable gate array(FPGA), a programmable logic array, a microprocessor, an application-specific integrated circuit (ASIC),, or any other device or combination of devices known to one of ordinary skill in the art that is capable of responding to and executing instructions in a defined manner to achieve a desired result. The processor may denote a type of a computational circuit, such as, for example, a microprocessor, a microcontroller, a complex instruction set computing (CISC) microprocessor, a reduced instruction set computing (RISC) microprocessor, a very long instruction word (VLIW) microprocessor, an explicitly parallel instruction computing (EPIC) microprocessor, a graphic processor, a digital signal processor, or a processing circuit of a different type. In one example, a processor or computer includes, or is connected to, one or more memories storing instructions or software that are executed by the processor or computer. Hardware components implemented by a processor or computer execute instructions or software, such as an operating system (OS) and one or more software applications that run on the OS, to perform the operations described herein. The hardware components also access, manipulate, process, create, and store data in response to execution of the instructions or software. For simplicity, the singular term "processor" or "computer" may be used in the description of the examples described herein, but in other examples multiple processors or computers are used, or a processor or computer includes multiple processing elements, or multiple types of processing elements, or both. In one example, a hardware component includes multiple processors, and in another example, a hardware component includes a processor and a controller. A hardware component has any one or more of different processing configurations, examples of which include a single processor, independent processors, parallel processors, single-instruction single-data (SISD) multiprocessing, single-instruction multiple-data (SIMD) multiprocessing, multiple-instruction single-data (MISD) multiprocessing, and multiple-instruction multiple-data (MIMD) multiprocessing.

The methods illustrated in FIGS. 2-3 and 7 that perform the operations described herein are performed by a processor or a computer as described above executing instructions or software to perform the operations described herein.

Instructions or software to control a processor or computer to implement the hardware components and perform the methods as described above are written as computer programs, code segments, instructions or any combination thereof, for individually or collectively instructing or configuring the processor or computer to operate as a machine or special-purpose computer to perform the operations performed by the hardware components and the methods as described above. In one example, the instructions or software include machine code that is directly executed by the processor or computer, such as machine code produced by a compiler. In another example, the instructions or software include higher-level code that is executed by the processor or computer using an interpreter. Programmers of ordinary skill in the art can readily write the instructions or software based on the block diagrams and the flow charts illustrated in the drawings and the corresponding descriptions in the specification, which disclose algorithms for performing the operations performed by the hardware components and the methods as described above.

The instructions or software to control a processor or computer to implement the hardware components and perform the methods as described above, and any associated data, data files, and data structures, are recorded, stored, or fixed in or on one or more non-transitory computer-readable storage media. Examples of a non-transitory computer-readable storage medium include read-only memory (ROM), random-access memory (RAM), flash memory, CD-ROMs, CD-Rs, CD+Rs, CD-RWs, CD+RWs, DVD-ROMs, DVD-Rs, DVD+Rs, DVD-RWs, DVD+RWs, DVD-RAMs, BD-ROMs, BD-Rs, BD-R LTHs, BD-REs, magnetic tapes, floppy disks, magneto-optical data storage devices, optical data storage devices, hard disks, solid-state disks, and any device known to one of ordinary skill in the art that is capable of storing the instructions or software and any associated data, data files, and data structures in a non-transitory manner and providing the instructions or software and any associated data, data files, and data structures to a processor or computer so that the processor or computer can execute the instructions. In one example, the instructions or software and any associated data, data files, and data structures are distributed over network-coupled computer systems so that the instructions and software and any associated data, data files, and data structures are stored, accessed, and executed in a distributed fashion by the processor or computer.

While this disclosure includes specific examples, it will be apparent to one of ordinary skill in the art that various changes in form and details may be made in these examples without departing from the scope of the claims. The examples described herein are to be considered in a descriptive sense only, and not for purposes of limitation. Descriptions of features or aspects in each example are to be considered as being applicable to similar features or aspects in other examples. Suitable results may be achieved if the described techniques are performed in a different order, and/or if components in a described system, architecture, device, or circuit are combined in a different manner, and/or replaced or supplemented by other components or their equivalents. Therefore, the scope of the disclosure is defined not by the detailed description, but by the claims.

**Claims**

1. A computer-method of extracting a feature of a biosignal, the method comprising:

   sensing (710) a waveform of a blood pulse wave;
   determining (720) an acceleration waveform (330) from the sensed waveform of the blood pulse wave by performing a second order differential on the sensed waveform;
   searching for a point ($T_0$) at which the acceleration waveform (330) is maximized in a vicinity of a start point of the acceleration waveform (330); and searching for a position of a positive peak point ($T_1$) positioned subsequent to the maximum point ($T_0$) of the acceleration waveform (330), wherein the start point corresponds to a position of the acceleration waveform (330) at which a heart contraction starts;
   **characterized in that** the method further comprises the steps of:

   extracting (730) an incident acceleration waveform (410) by extracting an interval from the acceleration waveform (330) from the maximum point ($T_0$) of the acceleration waveform to the subsequent positive peak point ($T_1$);
   obtaining a cross-correlation function (510) between the determined acceleration waveform (330) and the extracted incident acceleration waveform (410) over time by moving the incident acceleration waveform (410) during an interval of the acceleration waveform (330) from the start point ($T_0$) to an end point ($T_2$) subsequently located to said peak point ($T_1$);
   extracting a feature of the biosignal based on the cross-correlation between the acceleration waveform (330) and the incident acceleration waveform (410) by extracting the feature of the blood pulse wave based on at least one of a number of peak points comprised in the cross-correlation, positions of the peak points comprised in the cross-correlation, or values of the peak points comprised in the cross-correlation.

**2.** The method of claim 1, wherein the determining of the acceleration waveform (330) comprises determining the acceleration waveform based on an n-th sample value of the waveform, a (n-d)-th sample value of the waveform, and a (n+d)-th sample value of the waveform, wherein the d is a sample difference and the n is a positive integer greater than or equal to the d.

**3.** The method of claim 1, further comprising at least one of:

acquiring a periodic waveform of the blood pulse wave; or
acquiring an average waveform of the blood pulse wave.

**4.** The method of one of claims 1 to 3, wherein the incident acceleration waveform (410) is an interval waveform corresponding to a pressure interval generating the biosignal.

**5.** The method of one of claims 1 to 4, wherein the incident acceleration waveform (410) is an interval waveform corresponding to an interval during which an influence of a noise is lesser than a pulse wave signal generated by a heart contraction.

**6.** The method of one of claims 1 to 5, wherein the biosignal comprises a progressive wave starting from the heart and moving toward a body end portion and a reflective wave returned from the body end portion, and the extracting of the feature of the biosignal comprises searching for an initial positive peak point among positive peak points greater than or equal to a degree of correlation in the cross-correlation and extracting the initial positive peak point as a start point of the reflective wave.

**7.** A non-transitory computer readable medium comprising a program to control a processor to perform the method of one of claims 1 to 6.

**8.** An apparatus for extracting a feature of a biosignal, the apparatus comprising:

a sensor (910) configured to sense a blood pulse wave; and
a processor (920) configured to determine (720) an acceleration waveform (330) from the sensed waveform of the blood pulse wave by performing a second order differential on the sensed waveform, to search for a point $(T_0)$ at which the acceleration waveform (330) is maximized in a vicinity of a start point of the acceleration waveform (330), wherein the start point corresponds to a position of the acceleration waveform (330) at which a heart contraction starts; and to search for a position of a positive peak point $(T_1)$ positioned subsequent to the maximum point $(T_0)$ of the acceleration waveform (330),
**characterized in that** the processor (920) is further configured to extract (730) an incident acceleration waveform (410) by extracting an interval from the acceleration waveform (330) from the maximum point $(T_0)$ of the acceleration waveform to the subsequent positive peak point $(T_1)$, to obtain a cross-correlation function (510) between the determined acceleration waveform (330) and the extracted incident acceleration waveform (410) over time by moving the incident acceleration waveform (410) during an interval of the acceleration waveform (330) from the start point $(T_0)$ to an end point $(T_2)$ subsequently located to said peak point $(T_1)$, to extract a feature of the biosignal based on the cross-correlation between the acceleration waveform (330) and the incident acceleration waveform (410) by extracting the feature of the blood pulse wave based on at least one of a number of peak points comprised in the cross-correlation, positions of the peak points comprised in the cross-correlation, or values of the peak points comprised in the cross-correlation.

**9.** The apparatus of claim 8, adapted to operate according to one of claims 1 to 6.

**Patentansprüche**

**1.** Computerverfahren zum Extrahieren eines Merkmals von einem Biosignal, wobei das Verfahren umfasst:

Abtasten (710) einer Wellenform einer Blutpulswelle;
Bestimmen (720) einer Beschleunigungswellenform (330) aus der abgetasteten Wellenform der Blutpulswelle durch Durchführen eines Differentials zweiter Ordnung an der abgetasteten Wellenform;
Suchen nach einem Punkt $(T_0)$, an dem die Beschleunigungswellenform (330) in der Nähe eines Startpunktes der Beschleunigungswellenform (330) maximiert ist; und Suchen nach einer Position eines positiven Spitzen-

punktes ($T_1$), der nach dem Maximalpunkt ($T_0$) der Beschleunigungswellenform (330) positioniert ist, wobei der Startpunkt einer Position der Beschleunigungswellenform (330) entspricht, an der eine Herzkontraktion beginnt; **dadurch gekennzeichnet, dass** das Verfahren ferner die folgenden Schritte umfasst:

Extrahieren (730) einer einfallenden Beschleunigungswellenform (410) durch Extrahieren eines Intervalls aus der Beschleunigungswellenform (330) vom Maximalpunkt ($T_0$) der Beschleunigungswellenform bis zum nachfolgenden positiven Spitzenpunkt ($T_1$);

Erhalten einer Kreuzkorrelationsfunktion (510) zwischen der bestimmten Beschleunigungswellenform (330) und der extrahierten einfallenden Beschleunigungswellenform (410) über die Zeit durch Bewegen der einfallenden Beschleunigungswellenform (410) während eines Intervalls der Beschleunigungswellenform (330) von dem Startpunkt ($T_0$) zu einem Endpunkt ($T_2$), der sich nachfolgend zu dem Spitzenpunkt ($T_1$) befindet;

Extrahieren eines Merkmals von dem Biosignal auf der Grundlage der Kreuzkorrelation zwischen der Beschleunigungswellenform (330) und der einfallenden Beschleunigungswellenform (410) durch Extrahieren des Merkmals der Blutpulswelle auf der Grundlage von mindestens einem von einer Anzahl von Spitzenpunkten, die in der Kreuzkorrelation enthalten sind, Positionen der Spitzenpunkte, die in der Kreuzkorrelation enthalten sind, oder Werten der Spitzenpunkte, die in der Kreuzkorrelation enthalten sind.

2. Verfahren nach Anspruch 1, wobei die Bestimmung der Beschleunigungswellenform (330) die Bestimmung der Beschleunigungswellenform auf der Grundlage eines n-ten Abtastwertes der Wellenform, eines (n-d)-ten Abtastwertes der Wellenform und eines (n+d)-ten Abtastwertes der Wellenform umfasst, wobei d eine Abtastwertdifferenz und n eine positive ganze Zahl größer oder gleich d ist.

3. Verfahren nach Anspruch 1, ferner umfassend mindestens eines von:

Erfassen einer periodischen Wellenform der Blutpulswelle; oder
Erfassen einer durchschnittlichen Wellenform der Blutpulswelle.

4. Verfahren nach einem der Ansprüche 1 bis 3, wobei die einfallende Beschleunigungswellenform (410) eine Intervallwellenform ist, die einem Druckintervall entspricht, das das Biosignal erzeugt.

5. Verfahren nach einem der Ansprüche 1 bis 4, wobei die einfallende Beschleunigungswellenform (410) eine Intervallwellenform ist, die einem Intervall entspricht, während dessen ein Einfluss eines Störgeräusches geringer ist als ein Pulswellensignal, das durch eine Herzkontraktion erzeugt wird.

6. Verfahren nach einem der Ansprüche 1 bis 5, wobei das Biosignal eine fortschreitende Welle, die vom Herzen ausgeht und sich in Richtung eines Körperendabschnitts bewegt, und eine reflektierte Welle, die vom Körperendabschnitt zurückkehrt, umfasst, und das Extrahieren des Merkmals des Biosignals das Suchen nach einem anfänglichen positiven Spitzenpunkt unter positiven Spitzenpunkten, die größer oder gleich einem Korrelationsgrad in der Kreuzkorrelation sind, und das Extrahieren des anfänglichen positiven Spitzenpunkts als Startpunkt der reflektierenden Welle umfasst.

7. Nichtflüchtiges computerlesbares Medium umfassend ein Programm zur Steuerung eines Prozessors zum Durchführen des Verfahrens nach einem der Ansprüche 1 bis 6.

8. Vorrichtung zum Extrahieren eines Merkmals von einem Biosignal, wobei die Vorrichtung umfasst:

einen Sensor (910), konfiguriert zum Abtasten einer Blutpulswelle; und
einen Prozessor (920), konfiguriert zum Bestimmen (720) einer Beschleunigungswellenform (330) aus der abgetasteten Wellenform der Blutpulswelle durch Durchführen eines Differentials zweiter Ordnung an der abgetasteten Wellenform, um nach einem Punkt ($T_0$) zu suchen, an dem die Beschleunigungswellenform (330) in der Nähe eines Startpunktes der Beschleunigungswellenform (330) maximiert ist, wobei der Startpunkt einer Position der Beschleunigungswellenform (330) entspricht, an der eine Herzkontraktion beginnt; und um nach einer Position eines positiven Spitzenpunktes ($T_1$) zu suchen, der nach dem Maximalpunkt ($T_0$) der Beschleunigungswellenform (330) positioniert ist,

**dadurch gekennzeichnet, dass** der Prozessor (920) ferner konfiguriert ist, um eine einfallende Beschleunigungswellenform (410) zu extrahieren (730), indem ein Intervall aus der Beschleunigungswellenform (330) von dem Maximalpunkt ($T_0$) der Beschleunigungswellenform zu dem nachfolgenden positiven Spitzenpunkt ($T_1$)

extrahiert wird; um eine Kreuzkorrelationsfunktion (510) zwischen der bestimmten Beschleunigungswellenform (330) und der extrahierten einfallenden Beschleunigungswellenform (410) über die Zeit zu erhalten, indem die einfallende Beschleunigungswellenform (410) während eines Intervalls der Beschleunigungswellenform (330) von dem Startpunkt ($T_0$) zu einem Endpunkt ($T_2$) bewegt wird, der sich nachfolgend zu dem Spitzenpunkt ($T_1$) befindet, um ein Merkmal von dem Biosignal auf der Grundlage der Kreuzkorrelation zwischen der Beschleunigungswellenform (330) und der einfallenden Beschleunigungswellenform (410) zu extrahieren, indem das Merkmal der Blutpulswelle auf der Grundlage von mindestens einem von einer Anzahl von Spitzenpunkten, die in der Kreuzkorrelation enthalten sind, Positionen der Spitzenpunkte, die in der Kreuzkorrelation enthalten sind, oder Werten der Spitzenpunkte, die in der Kreuzkorrelation enthalten sind, extrahiert wird.

9. Vorrichtung nach Anspruch 8, angepasst zum Betrieb nach einem der Ansprüche 1 bis 6.

**Revendications**

1. Procédé informatique d'extraction d'une caractéristique d'un biosignal, le procédé comprenant :

la détection (710) d'une forme d'onde d'une onde de pouls ;
la détermination (720) d'une forme d'onde d'accélération (330) à partir de la forme d'onde détectée de l'onde de pouls en effectuant une différentielle de second ordre sur la forme d'onde détectée ;
la recherche d'un point ($T_0$) auquel la forme d'onde d'accélération (330) est maximisée à proximité d'un point de départ de la forme d'onde d'accélération (330) ; et la recherche d'une position d'un point pic ($T_1$) positif positionné après le point maximal ($T_0$) de la forme d'onde d'accélération (330), le point de départ correspondant à une position de la forme d'onde d'accélération (330) à laquelle commence une contraction du cœur ;
**caractérisé en ce que** le procédé comprend en outre les étapes de :

extraction (730) d'une forme d'onde d'accélération incidente (410) par extraction d'un intervalle depuis la forme d'onde d'accélération (330) depuis le point maximal ($T_0$) de la forme d'onde d'accélération vers le point pic positif ($T_1$) ultérieur ;
obtention d'une fonction de corrélation croisée (510) entre la forme d'onde d'accélération (330) déterminée et la forme d'onde d'accélération incidente (410) extraite dans le temps en déplaçant la forme d'onde d'accélération incidente (410) durant un intervalle de la forme d'onde d'accélération (330) depuis le point de départ ($T_0$) vers un point final ($T_2$) localisé après ledit point pic ($T_1$) ;
extraction d'une caractéristique du biosignal sur la base de la corrélation croisée entre la forme d'onde d'accélération (330) et la forme d'onde d'accélération incidente (410) par extraction de la caractéristique de l'onde de pouls sur la base d'au moins un nombre des points pics compris dans la corrélation croisée, des positions des points pics compris dans la corrélation croisée, ou des valeurs des points pics compris dans la corrélation croisée.

2. Procédé selon la revendication 1, la détermination de la forme d'onde d'accélération (330) comprenant la détermination la forme d'onde d'accélération sur la base d'une n-ième valeur d'échantillon de la forme d'onde, d'une (n-d)-ième valeur d'échantillon de la forme d'onde, et d'une (n+d)-ième valeur d'échantillon de la forme d'onde, le d étant une différence d'échantillon et le n étant un nombre entier positif supérieur ou égal à d.

3. Procédé selon la revendication 1, comprenant en outre au moins l'une parmi :

l'acquisition d'une forme d'onde périodique de l'onde de pouls ; ou
l'acquisition d'une forme d'onde moyenne de l'onde de pouls.

4. Procédé selon l'une des revendications 1 à 3, la forme d'onde d'accélération incidente (410) étant une forme d'onde d'intervalle correspondant à un intervalle de pression générant le biosignal.

5. Procédé selon l'une des revendications 1 à 4, la forme d'onde d'accélération incidente (410) étant une forme d'onde d'intervalle correspondant à un intervalle durant lequel une influence d'un bruit est inférieure à un signal d'onde de pouls généré par une contraction du cœur.

6. Procédé selon l'une des revendications 1 à 5, le biosignal comprenant une onde progressive commençant depuis le cœur et se déplaçant vers une portion d'extrémité du corps et une onde de réflexion renvoyée de la portion d'extrémité du corps, et l'extraction de la caractéristique du biosignal comprenant la recherche d'un point pic positif initial parmi les points pics positifs supérieurs ou égaux à un degré de corrélation dans la corrélation croisée et l'extraction du point pic positif initial comme point de départ de l'onde de réflexion.

7. Milieu non transitoire pouvant être lu par ordinateur comprenant un programme de commande d'un processeur pour effectuer le procédé selon l'une des revendications 1 à 6.

8. Appareil d'extraction d'une caractéristique d'un biosignal, l'appareil comprenant :

un capteur (910) configuré pour détecter une onde de pouls ; et
un processeur (920) configuré pour déterminer (720) une forme d'onde d'accélération (330) à partir de la forme d'onde détectée de l'onde de pouls en effectuant une différentielle de second ordre sur la forme d'onde détectée, pour chercher un point ($T_0$) auquel la forme d'onde d'accélération (330) est maximisée à proximité d'un point de départ de la forme d'onde d'accélération (330), le point de départ correspondant à une position de la forme d'onde d'accélération (330) à laquelle commence une contraction du cœur ; et pour chercher une position d'un point pic ($T_1$) positif positionné après le point maximal ($T_0$) de la forme d'onde d'accélération (330), **caractérisé en ce que** le processeur (920) est en outre configuré pour extraire (730) une forme d'onde d'accélération incidente (410) par extraction d'un intervalle depuis la forme d'onde d'accélération (330) depuis le point maximal ($T_0$) de la forme d'onde d'accélération vers le point pic ($T_1$) positif ultérieur, afin d'obtenir une fonction de corrélation croisée (510) entre la forme d'onde d'accélération (330) déterminée et la forme d'onde d'accélération incidente (410) extraite dans le temps en déplaçant la forme d'onde d'accélération incidente (410) durant un intervalle de la forme d'onde d'accélération (330) depuis le point de départ ($T_0$) vers un point final ($T_2$) localisé par la suite au niveau dudit point pic ($T_1$), pour extraire une caractéristique du biosignal sur la base de la corrélation croisée entre la forme d'onde d'accélération (330) et la forme d'onde d'accélération incidente (410) par extraction de la caractéristique de l'onde de pouls sur la base d'au moins un nombre des points pics compris dans la corrélation croisée, des positions des points pics compris dans la corrélation croisée, ou des valeurs des points pics compris dans la corrélation croisée.

9. Appareil selon la revendication 8, adapté pour fonctionner selon l'une des revendications 1 à 6.

FIG. 1

110

PULSE VOLUME

X

Y

TIME

130

PULSE VOLUME

TIME

**FIG. 2**

START

↓

CALCULATE ACCELERATION WAVEFORM FROM
WAVEFORM OF BIOSIGNAL SENSED FROM USER — 210

↓

EXTRACT INCIDENT ACCELERATION WAVEFORM
FROM ACCELERATION WAVEFORM — 220

↓

EXTRACT FEATURE OF BIOSIGNAL BASED ON
CROSS-CORRELATION BETWEEN ACCELERATION
WAVEFORM AND INCIDENT ACCELERATION WAVEFORM — 230

↓

END

**FIG. 3**

310

QUADRATIC DIFFERENTIAL

330

FIG. 4

FIG. 5

EP 3 165 155 B1

## FIG. 6

## FIG. 7

START

710
ACQUIRE WAVEFORM OF BIOSIGNAL

720
CALCULATE ACCELERATION WAVEFORM BY PERFORMING
QUADRATIC DIFFERENTIAL ON WAVEFORM OF BIOSIGNAL

730
EXTRACT INCIDENT ACCELERATION WAVEFORM FROM
INTERVAL WAVEFORM FROM MAXIMUM POINT OF
ACCELERATION WAVEFORM TO SUBSEQUENT
POSITIVE PEAK POINT

740
CALCULATE DEGREE OF CORRELATION BETWEEN
INCIDENT ACCELERATION WAVEFORM AND
ACCELERATION WAVEFORM OVER TIME WHILE MOVING
INCIDENT ACCELERATION WAVEFORM DURING
INTERVAL OF ACCELERATION WAVEFORM

750
SEARCH FOR INITIAL POSITIVE PEAK POINT AMONG
POSITIVE PEAK POINTS GREATER THAN OR EQUAL TO
PREDETERMINED DEGREE OF CORRELATION

760
EXTRACT FOUND POSITIVE PEAK POINT AS START
POINT OF REFLECTIVE WAVE

END

**FIG. 8**

**FIG. 9**

900

940

910
SENSOR

950
DISPLAY

920
PROCESSOR

930
MEMORY

**EP 3 165 155 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20140073968 A1
- US 20130079656 A1
- US 20060247542 A1